# EUROPÄISCHE PATENTSCHRIFT

(11) **EP 1 060 734 B1**
(45) Veröffentlichungstag und Bekanntmachung des Hinweises auf die Patenterteilung: **13.09.2006**
(21) Anmeldenummer: 00110272.2
(22) Anmeldetag: 22.05.2000
(51) Int. Cl.: A61K 8/49, A61Q 17/04, C08K 5/47, C08K 5/3447, C08K 5/35, C07D 235/16, C07D 263/56, C07D 277/64

(54) **Verwendung von cyclischen Enaminen als Lichtschutzmittel**
Use of cyclic enamines as sun protection agents
Utilisation d' enamines cycliques comme agents photoprotecteurs

(30) Priorität: 18.06.1999 DE 19928033
(43) Veröffentlichungstag der Anmeldung: 20.12.2000
(73) Patentinhaber: BASF Aktiengesellschaft, 67056 Ludwigshafen (DE)
(72) Erfinder: Habeck, Thorsten, Dr., 67149 Meckenheim (DE); Prechtl, Frank, Dr., 60318 Frankfurt (DE); Wünsch, Thomas, Dr., 67346 Speyer (DE); Westenfelder, Horst, 67435 Neustadt (DE)

(56) Entgegenhaltungen:
- EP-A- 0 349 139
- WO-A-91/11989
- R. SAALFRANK ET AL: CHEMISCHE BERICHTE , Bd. 122, Nr. 5, 1989, Seiten 969-973, XP002231576
- F. CADO ET AL: BULL SOC. CHIM. FR., Bd. 133, Nr. 6, 1996, Seiten 587-595, XP001109667
- W.D. RUDORF ET AL: JOURNAL FÜR PRAKTISCHE CHEMIE, Bd. 319, Nr. 4, 1977, Seiten 545-560, XP001091200

## Beschreibung

Die Erfindung betrifft die Verwendung von bicyclischen und tricyclischen Enaminen, die an der En-Doppelbindung elektronenziehende Substituenten tragen als Lichtschutzmittel in kosmetischen und pharmazeutischen Zubereitungen und als Lichtschutzzusätze in Kunststoffen. Die Erfindung betrifft ferner neue lichtschutzwirksame Verbindungen der genannten Art.

Die in kosmetischen und pharmazeutischen Zubereitungen eingesetzten Lichtschutzmittel haben die Aufgabe, schädigende Einflüsse des Sonnenlichts auf die menschliche Haut zu verhindern oder zumindest in ihren Auswirkungen zu reduzieren. Daneben dienen diese Lichtschutzmittel aber auch dem Schutz weiterer Inhaltsstoffe vor Zerstörung oder Abbau durch UV-Strahlung. In haarkosmetischen Formulierungen soll eine Schädigung der Keratinfaser durch UV-Strahlen vermindert werden.

Das an die Erdoberfläche gelangende Sonnenlicht hat einen Anteil an UV-B- (280 bis 320 nm) und an UV-A-Strahlung (> 320 nm), welche sich direkt an den Bereich des sichtbaren Lichtes anschließen. Der Einfluß auf die menschliche Haut macht sich besonders bei der UV-B-Strahlung durch Sonnenbrand bemerkbar. Dementsprechend bietet die Industrie eine größere Zahl von Substanzen an, welche die UV-B-Strahlung absorbieren und damit den Sonnenbrand verhindern.

Nun haben dermatologische Untersuchungen gezeigt, daß auch die UV-A-Strahlung durchaus Hautschädigungen und Allergien hervorrufen kann, indem beispielsweise das Keratin oder Elastin geschädigt wird. Hierdurch werden Elastizität und Wasserspeichervermögen der Haut reduziert, d.h. die Haut wird weniger geschmeidig und neigt zur Faltenbildung. Die auffallend hohe Hautkrebshäufigkeit in Gegenden starker Sonneneinstrahlung zeigt, daß offenbar auch Schädigungen der Erbinformationen in den Zellen durch Sonnenlicht, speziell durch UV-A-Strahlung, hervorgerufen werden. All diese Erkenntnisse lassen daher die Entwicklung effizienter Filtersubstanzen für den UV-A-Bereich notwendig erscheinen.

Es besteht ein wachsender Bedarf an Lichtschutzmitteln für kosmetische und pharmazeutische Zubereitungen, die vor allem als UV-A-Filter dienen können und deren Absorptionsmaxima deshalb im Bereich von ca. 320 bis 380 nm liegen sollten. Um mit einer möglichst geringen Einsatzmenge die gewünschte Wirkung zu erzielen, sollten derartige Lichtschutzmittel zusätzlich eine hoch spezifische Extinktion aufweisen. Außerdem müssen Lichtschutzmittel für kosmetische Präparate noch eine Vielzahl weiterer Anforderungen erfüllen, beispielsweise gute Löslichkeit in kosmetischen Ölen, hohe Stabilität der mit ihnen hergestellten Emulsionen, toxikologische Unbedenklichkeit sowie geringen Eigengeruch und geringe Eigenfärbung.

Eine weitere Anforderung, der Lichtschutzmittel genügen müssen, ist eine ausreichende Photostabilität. Dies ist aber mit den bisher verfügbaren UV-A absorbierenden Lichtschutzmitteln nicht oder nur unzureichend gewährleistet.

In der französischen Patentschrift Nr. 2 440 933 wird das 4-(1,1-Dimethylethyl)-4'-methoxydibenzoylmethan als UV-A-Filter beschrieben. Es wird vorgeschlagen, diesen speziellen UV-A-Filter, der von der Firma GIVAUDAN unter der Bezeichnung "PARSOL 1789" verkauft wird, mit verschiedenen UV-B-Filtern zu kombinieren, um die gesamten UV-Strahlen mit einer Wellenlänge von 280 bis 380 nm zu absorbieren.

Dieser UV-A-Filter ist jedoch, wenn er allein oder in Kombination mit UV-B-Filtern verwendet wird, photochemisch nicht beständig genug, um einen anhaltenden Schutz der Haut während eines längeren Sonnenbades zu gewährleisten, was wiederholte Anwendungen in regelmäßigen und kurzen Abständen erfordert, wenn man einen wirksamen Schutz der Haut gegen die gesamten UV-Strahlen erzielen möchte.

Deshalb sollen gemäß EP-A-0 514 491 die nicht ausreichend photostabilen UV-A-Filter durch den Zusatz von 2-Cyan-3,3-diphenylacrylsäureestern stabilisiert werden, die selbst im UV-B-Bereich als Filter dienen.

Weiterhin wurde gemäß EP-A-0 251 398 schon vorgeschlagen, UV-A- und UV-B-Strahlung absorbierende Chromophore durch ein Bindeglied in einem Molekül zu vereinen. Dies hat den Nachteil, daß einerseits keine freie Kombination von UV-A- und UV-B-Filtern in der kosmetischen Zubereitung mehr möglich ist und daß Schwierigkeiten bei der chemischen Verknüpfung der Chromophore nur bestimmte Kombinationen zulassen.

US 4,950,467 beschreibt die Verwendung von 2,4-Pentadiensäurederivaten als UV-Absorber in kosmetischen Präparaten. Die in dieser Patentschrift bevorzugt genannten Monoaryl-substituierten Verbindungen haben ebenfalls den Nachteil, daß sie nicht genügend photostabil sind.

Es bestand daher die Aufgabe, Lichtschutzmittel für kosmetische und pharmazeutische Zwecke vorzuschlagen, die vor allem im UV-A-Bereich (und gegebenenfalls alternativ im UV-B-Bereich) mit hoher Extinktion absorbieren, die photostabil sind, eine geringe Eigenfarbe d.h. eine scharfe Bandenstruktur aufweisen und je nach Substituent in Öl oder Wasser löslich sind.

Diese Aufgabe wurde erfindungsgemäß gelöst durch die Verwendung von Verbindungen der Formel I 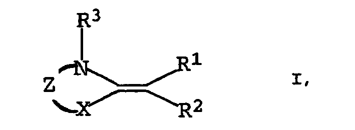 in der
R¹ und R² gleiche oder verschiedene, Elektronen anziehende Reste, ausgewählt aus der Gruppe bestehend aus Cyan, Alkyl- oder Arylcarbonyl, Alkyloxy- oder Aryloxycarbonyl und gegebenenfalls substituiertem Aminocarbonyl,
- R³: ein Wasserstoffatom, einen C₁-C₂₀-Alkylrest oder C₃-C₂₀ Cycloalkylrest oder einen Rest der Formel -CH₂-CH₂-SO₃⁻ M⁺ bedeutet, wobei M⁺ für ein Kation steht,
- X: den zweiwertigen Rest des Sauerstoffs, Schwefels oder den Rest 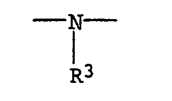 bedeutet, wobei R³ die oben genannte Bedeutung hat,
- Z: die zweiwertigen Reste der Formeln II oder III bedeutet, die mit dem Rest der Formel I ein anelliertes System bilden 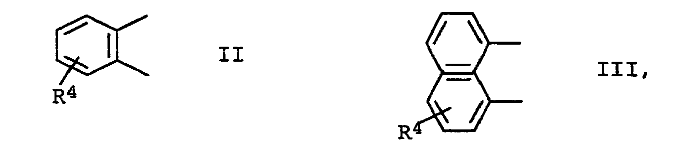 wobei R⁴ ein oder mehrfach an den Benzyliden- I bzw. Naphthylidenring II gebunden sein kann und Wasserstoff, Alkyl, Cycloalkyl, Alkyloxy, Cycloalkyloxy, Alkoxycarbonyl, Mono- oder Dialkylaminocarbonyl, Alkylamino, Dialkylamino, alle mit jeweils bis zu 20 C-Atomen, sowie Cyan, Amino und SO₃-M⁺ bedeuten, wobei M⁺ für ein Metallkation steht,
als photostabile UV-Filter in kosmetischen und pharmazeutischen Zubereitungen zum Schutz der menschlichen Haut und menschlicher Haare gegen Sonnenstrahlen, allein oder zusammen mit an sich für kosmetischen und pharmazeutischen Zubereitungen bekannten, im UV-Bereich absorbierenden Verbindungen.

Dabei sind solche Verbindungen bevorzugt, bei denen Z den Rest der Formel II bedeutet.

Bezüglich der Substituenten werden bevorzugt solche Verbindungen verwendet, bei denen R¹ Cyan oder Alkyloxycarbonyl mit 2 bis 12 C-Atomen, R² Wasserstoff, R³ Wasserstoff oder Methyl und R⁴ C₁-C₈ Alkyl, C₁-C₈-Alkoxy oder SO₃⁻M⁺ bedeuten, wobei M⁺ für ein Kation steht, ausgewählt aus der Gruppe bestehend aus 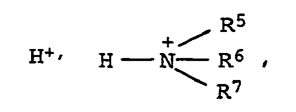 wobei R⁵, R⁶ und R⁷ Wasserstoff oder niedermolekulares Alkyl oder Hydroxyalkyl bedeuten, und Metallkationen.

Die Alkylreste in den Substituenten R¹ bis R⁴ haben vorzugsweise 1 bis 8 C-Atome und sind insbesondere niedermolekulare Alkylreste mit 1 bis 5 C-Atomen und vorzugsweise Methylreste.

Die Alkylreste in den Substituenten R⁴ sind isocyclische oder heterocyclische Reste mit 5 bis 10 Ringatomen und 3 bis 5 C-Atomen, die weiter substituiert sein können.

In den Resten -SO₃⁻M⁺ und -CH₂-CH₂-SO₃⁻M⁺ ist M⁺ in der Regel ein Alkali-, Erdalkal- oder Ammoniumkation, insbesondere Na⁺, K⁺, NH₄⁺, ⁺NH(C₂H₅)₃ und ⁺NH(-CH₂-CH₂OH)₃. Allgemein gilt, daß nur solche Kationen in Betracht kommen, die bei den Anwendungskonzentrationen physiologisch verträglich sind.

Von den Verbindungen der Formel I sind die Verbindungen der Formel V neu 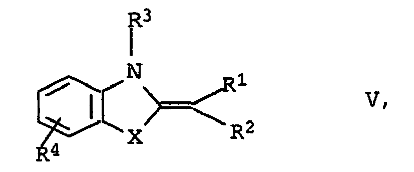 in der X den zweiwertigen Rest des Sauerstoffs, Schwefels oder den Rest 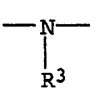 bedeutet, und R¹ und R² unabhängig voneinander CN oder Alkoxycarbonyl mit 2 bis 10 C-Atomen, das durch einen Rest -CH₂)ₙ-SO₃⁻M⁺ substituiert sein kann, R³ Wasserstoff, Methyl oder -(CH₂)ₙ-SO₃⁻M⁺ und R⁴ Wasserstoff oder einen oder mehrere gleiche oder verschiedene Reste C₁- bis C₈-Alkyl, C₁- bis C₈-Alkoxy oder den Rest -SO₃⁻M⁺ bedeutet, wobei jeweils n die Zahlen 2 oder 3 und M⁺ ein Kation bedeutet, ausgewählt aus der Gruppe bestehend aus 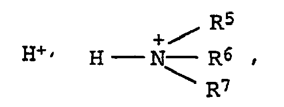 in der R⁵, R⁶ und R⁷ für Wasserstoff oder niedermolekulares Alkyl oder Hydroxyalkyl steht und Metallkationen,
mit der Maßgabe, daß die Verbindung der Formel V mindestens einen Rest -(CH₂)ₙSO₃⁻M⁺ oder -SO₃⁻M⁺ aufweist.

Die Verbindungen der Formel I werden in an sich bekannter Weise erhalten, wie dies z.B. in CH 601984 beschrieben ist, durch Umsetzung einer Verbindung der Formel VI 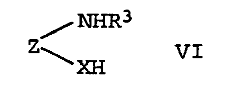 mit einer Verbindung der Formel VII 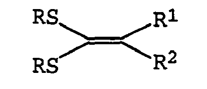 in denen R¹ bis R³ die oben genannten Bedeutungen haben und R einen niedermolekularen Alkylrest, vorzugsweise Methyl bedeutet.

Dies sei am Beispiel der Verbindung 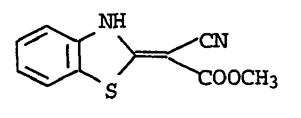 durch die folgende Reaktionsgleichung exemplifiziert.

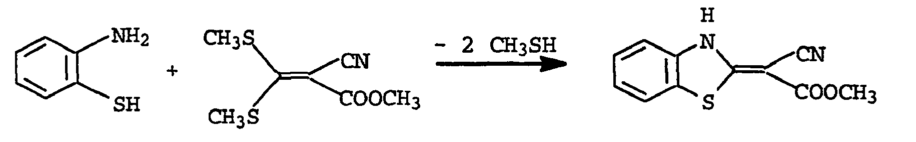

Die Umsetzung findet durch Erhitzen auf höhere Temperaturen, z.B. auf 20 bis 150°C, bevorzugt 50 bis 120°C und insbesondere 70 bis 100°C statt.

Gegenstand der vorliegenden Erfindung sind weiterhin kosmetische und pharmazeutische Zubereitungen, die 0,1 bis 10 Gew.-%, vorzugsweise 1 bis 7 Gew.-%, bezogen auf die gesamte Menge der kosmetischen und pharmazeutischen Zubereitung, eine oder mehrere der Verbindungen der Formel I zusammen mit an sich für kosmetische und pharmazeutische Zubereitungen bekannten, im UV-A- und UV-B-Bereich absorbierenden Verbindungen als Lichtschutzmittel enthalten, wobei die Verbindungen der Formel I in der Regel in geringerer Menge als die UV-B-absorbierenden Verbindungen eingesetzt werden.

Die Lichtschutzmittel enthaltenden kosmetischen und pharmazeutischen Zubereitungen sind in der Regel auf der Basis eines Trägers, der mindestens eine Ölphase enthält. Es sind aber auch Zubereitungen allein auf wäßriger Basis bei Verwendung von Verbindungen mit hydrophilen Substituenten möglich. Demgemäß kommen Öle, Öl-in-Wasser- und Wasser-in-Öl-Emulsionen, Cremes und Pasten, Lippenschutzstiftmassen oder fettfreie Gele in Betracht.

Solche Sonnenschutzpräparate können demgemäß in flüssiger, pastöser oder fester Form vorliegen, beispielsweise als Wasser-in-Öl-Cremes, Öl-in-Wasser-Cremes und -Lotionen, Aerosol-Schaumcremes, Gele, Öle, Fettstifte, Puder, Sprays oder alkoholisch-wäßrige Lotionen.

Als Emulsionen kommen u.a. auch O/W-Makroemulsionen, O/W-Mikroemulsionen oder O/W/O-Emulsionen mit in dispergierter Form vorliegenden Verbindungen der Formel I in Frage, wobei die Emulsionen durch Phaseninversionstechnologie, gemäß DE-A-197 26 121 erhältlich sind.

Übliche kosmetische Hilfsstoffe, die als Zusätze in Betracht kommen können, sind z.B. Co-Emulgatoren, Fette und Wachse, Stabilisatoren, Verdickungsmittel, biogene Wirkstoffe, Filmbildner, Duftstoffe, Farbstoffe, Perlglanzmittel, Konservierungsmittel, Pigmente, Elektrolyte (z.B. Magnesiumsulfat) und pH-Regulatoren. Als Co-Emulgatoren kommen vorzugsweise bekannte W/O- und daneben auch O/W-Emulgatoren wie etwa Polyglycerinester, Sorbitanester oder teilveresterte Glyceride in Betracht. Typische Beispiele für Fette sind Glyceride; als Wachse sind u.a. Bienenwachs, Paraffinwachs oder Mikrowachse gegebenenfalls in Kombination mit hydrophilen Wachsen zu nennen. Als Stabilisatoren können Metallsalze von Fettsäuren wie z.B. Magnesium-, Aluminium- und/oder Zinkstearat eingesetzt werden. Geeignete Verdickungsmittel sind beispielsweise vernetzte Polyacrylsäuren und deren Derivate, Polysaccharide, insbesondere Xanthan-Gum, Guar-Guar, Agar-Agar, Alginate und Tylosen, Carboxymethylcellulose und Hydroxyethylcellulose, ferner Fettalkohole, Monoglyceride und Fettsäuren, Polycrylate, Polyvinylalkohol und Polyvinylpyrrolidon. Unter biogenen Wirkstoffen sind beispielsweise Pflanzenextrakte, Eiweißhydrolysate und Vitaminkomplexe zu verstehen. Gebräuchliche Filmbildner sind beispielsweise Hydrocolloide wie Chitosan, mikrokristallines Chitosan oder quaterniertes Chitosan, Polyvinylpyrrolidon, Vinylpyrrolidon-Vinylacetat-Copolymerisate, Polymere der Acrylsäurereihe, quaternäre Cellulose-Derivate und ähnliche Verbindungen. Als Konservierungsmittel eignen sich beispielsweise Formaldehydlösung, p-Hydroxybenzoat oder Sorbinsäure. Als Perlglanzmittel kommen beispielsweise Glycoldistearinsäureester wie Ethylenglycoldistearat, aber auch Fettsäuren und Fettsäuremonoglycolester in Betracht. Als Farbstoffe können die für kosmetische Zwecke geeigneten und zugelassenen Substanzen verwendet werden, wie sie beispielsweise in der Publikation "Kosmetische Färbemittel" der Farbstoffkommission der Deutschen Forschungsgemeinschaft, veröffentlicht im Verlag Chemie, Weinheim, 1984, zusammengestellt sind. Diese Farbstoffe werden üblicherweise in Konzentration von 0,001 bis 0,1 Gew.-%, bezogen auf die gesamte Mischung, eingesetzt.

Ein zusätzlicher Gehalt an Antioxidantien ist im allgemeinen bevorzugt. So können als günstige Antioxidantien alle für kosmetische und/oder dermatologische Anwendungen geeigneten oder gebräuchlichen Antioxidantien verwendet werden.

Vorteilhafterweise werden die Antioxidantien gewählt aus der Gruppe, bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thiorodoxin, Glutathion, Cystein, Cystin, Cystamin und deren Glycosyl-, N-Acetyl-, Methyl-, Ethyl-, Propyl-, Amyl-, Butyl-, und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximine, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Biliburin, Biliverdin, EDTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und deren Derivate (z.B. Ascorbylpalmitat, Mg-Ascorbylphosphat, Ascorbylacetat), Tocopherol und Derivate (z.B. Vitamin-E-Acetat, Tocotrienol), Vitamin A und Derivate (Vitamin-A-Palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄), Selen und dessen Derivate (z.B. Selenmethionin), Stilbene und deren Derivate (z.B. Stilbenoxid, Trans-Stilbenoxid).

Die Menge der vorgenannten Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 1 bis 10 Gew.-%, bezogen auf das Gesamtgewicht der Zubereitung.

Sofern Vitamin E und/oder dessen Derivate das oder die Antioxidantien darstellen, ist es vorteilhaft, deren jeweilige Konzentration aus dem Bereich von 0,001 bis 10 Gew.-%, bezogen auf das Gesamtgewicht der Formulierung, zu wählen.

Sofern Vitamin A und/oder dessen Derivate bzw. Carotinoide das oder die Antioxidantien darstellen, ist es vorteilhaft, deren jeweilige Konzentration aus dem Bereich von 0,001 bis 10 Gew.-%, bezogen auf das Gesamtgewicht der Formulierung, zu wählen.

Übliche Ölkomponenten in der Kosmetik sind beispielsweise Paraffinöl, Glycerylstearat, Isopropylmyristat, Diisopropyladipat, 2-Ethylhexansäurecetylstearylester, hydriertes Polyisobuten, Vaseline, Caprylsäure/Caprinsäure-Triglyceride, mikrokristallines Wachs, Lanolin und Stearinsäure.

Der Gesamtanteil der Hilfs- und Zusatzstoffe kann 1 bis 80, vorzugsweise 6 bis 40 Gew.-% und der nicht wäßrige Anteil ("Aktivsubstanz") 20 bis 80, vorzugsweise 30 bis 70 Gew.-% - bezogen auf die Mittel - betragen. Die Herstellung der Mittel kann in an sich bekannter Weise, d.h. beispielsweise durch Heiß-, Kalt-, Heiß-Heiß/Kalt- bzw. PIT-Emulgierung erfolgen. Hierbei handelt es sich um ein rein mechanisches Verfahren, eine chemische Reaktion findet nicht statt.

Solche Sonnenschutzpräparate können demgemäß in flüssiger, pastöser oder fester Form vorliegen, beispielsweise als Wasser-in-Öl-Cremes, Öl-in-Wasser-Cremes und -Lotionen, Aerosol-Schaumcremes, Gele, Öle, Fettstifte, Puder, Sprays oder alkoholisch-wäßrige Lotionen.

Schließlich können weitere an sich bekannte im UV-Bereich absorbierenden Substanzen mitverwendet werden, sofern sie im Gesamtsystem der erfindungsgemäß zu verwendenden Kombination aus UV-Filtern stabil sind.

Der größte Teil der Lichtschutzmittel in den zum Schutz der menschlichen Epidermis dienenden kosmetischen und pharmazeutischen Zubereitungen besteht aus Verbindungen, die UV-Licht im UV-B-Bereich absorbieren d.h. im Bereich von 280 bis 320 nm. Beispielsweise beträgt der Anteil der erfindungsgemäß zu verwendenden UV-A-Absorber 10 bis 90 Gew.-%, bevorzugt 20 bis 50 Gew.-% bezogen auf die Gesamtmenge von UV-B und UV-A absorbierenden Substanzen.

Als UV-Filtersubstanzen, die in Kombination mit den erfindungsgemäß zu verwendenden Verbindungen der Formel I angewandt werden, kommen beliebige UV-A- und UV-B-Filtersubstanzen in Betracht. Beispielsweise sind zu nennen:

| Nr. | Stoff | CAS-Nr. (=Säure) |
|---|---|---|
| 1 | 4-Aminobenzoesäure | 150-13-0 |
| 2 | 3-(4'Trimethylammonium)-benzylidenbornan-2-on-methylsulfat | 52793-97-2 |
| 3 | 3,3,5-Trimethyl-cyclohexyl-salicylat (Homosalatum) | 118-56-9 |
| 4 | 2-Hydroxy-4-methoxy-benzophenon (Oxybenzonum) | 131-57-7 |
| 5 | 2-Phenylbenzimidazol-5-sulfonsäure und ihre Kalium-, Natrium- u. Triethanolaminsalze | 27503-81-7 |
| 6 | 3,3'-(1,4-Phenylendimethin)-bis(7,7-dimethyl-2-oxobicyclo[2.2.1]heptan-1-methansulfonsäure) und ihre Salze | 90457-82-2 |
| 7 | 4-Bis(polyethoxy)amino-benzoesäurepolyethoxyethylester | 113010-52-9 |
| 8 | 4-Dimethylamino-benzoesäure-2-ethylhexylester | 21245-02-3 |
| 9 | Salicylsäure-2-ethylhexylester | 118-60-5 |
| 10 | 4-Methoxy-zimtsäure-2-isoamylester | 71617-10-2 |
| 11 | 4-Methoxy-zimtsäure-2-ethylhexylester | 5466-77-3 |
| 12 | 2-Hydroxy-4-methoxy-benzophenon-5-sulfon-(Sulisobenzonum) und das Natriumsalz | 4065-45-6 |
| 13 | 3-(4'-Sulfo)benzyliden-bornan-2-on und Salze | 58030-58-6 |
| 14 | 3-Benzylidenbornan-2-on | 16087-24-8 |
| 15 | 1-(4'-Isopropylphenyl)-3-phenylpropan-1,3-dion | 63260-25-9 |
| 16 | 4-Isopropylbenzylsalicylat | 94134-93-7 |
| 17 | 2,4,6-Trianilin-(o-carbo-2'-ethylhexyl-1'-oxy)-1,3,5-triazin | 88122-99-0 |
| 18 | 3-Imidazol-4-yl-acrylsäure und ihr Ethylester | 104-98-3 |
| 19 | 2-Cyano-3,3-diphenylacrylsäureethylester | 5232-99-5 |
| 20 | 2-Cyano-3,3-diphenylacrylsäure-2'-ethylhexylester | 6197-30-4 |
| 21 | Menthyl-o-aminobenzoate oder: 5-Methyl-2-(1-methylethyl)-2-aminobenzoate | 134-09-8 |
| 22 | Glyceryl p-aminobenzoat oder: 4-Aminobenzoesäure-1-glyceryl-ester | 136-44-7 |
| 23 | 2,2'-Dihydroxy-4-methoxybenzophenon (Dioxybenzone) | 131-53-3 |
| 24 | 2-Hydroxy-4-methoxy-4-methylbenzophenon (Mexonon) | 1641-17-4 |
| 25 | Triethanolamin Salicylat | 2174-16-5 |
| 26 | Dimethoxyphenylglyoxalsäure oder: 3,4-dimethoxy-phenyl-glyoxal-saures Natrium | 4732-70-1 |
| 27 | 3-(4'Sulfo)benzyliden-bornan-2-on und seine Salze | 56039-58-8 |
| 28 | 4-tert.-Butyl-4'-methoxy-dibenzoylmethan | 70356-09-1 |
| 29 | 2,2',4,4'-Tetrahydroxybenzophenon | 131-55-5 |
| 30 | 2,2'-Methylen-bis-[6(2H-benzotriazol-2-yl)-4-(1,1,3,3,-tetramethylbutyl)phenol] | 103597-45-1 |
| 31 | 2,2'-(1,4-Phenylen)-bis-1H-benzimidazol-4,6-disulfonsäure, Na-Salz | 180898-37-7 |
| 32 | 2,4-bis-[4-(2-Ethylhexyloxy)-2-hydroxy]phenyl-6-(4-methoxyphenyl)-(1,3,5)-triazin | 187393-00-6 |

Die erfindungsgemäßen kosmetischen und dermatologischen Zubereitungen können vorteilhafterweise außerdem anorganische Pigmente auf Basis von Metalloxiden und/oder anderen in Wasser schwerlöslichen oder unlöslichen Metallverbindungen, insbesondere der Oxide des Titans (TiO₂), Zinks (ZnO), Eisens (z.B. Fe₂O₃), Zirkoniums (ZrO₂), Siliciums (SiO₂), Mangans (z.B. MnO), Aluminiums (Al₂O₃), Cers (z.B. Ce₂O₃), Mischoxiden der entsprechenden Metalle sowie Abmischungen aus solchen Oxiden enthalten. Besonders bevorzugt handelt es sich um Pigmente auf der Basis TiO₂ und ZnO.

Es ist besonders vorteilhaft im Sinne der vorliegenden Erfindung, wenngleich nicht zwingend, wenn die anorganischen Pigmente in hydrophober Form vorliegen, d.h. daß sie oberflächlich wasserabweisend behandelt sind. Diese Oberflächenbehandlung kann darin bestehen, daß die Pigmente nach an sich bekannter Weise, wie in DE-A-33 14 742 beschrieben, mit einer dünnen hydrophoben Schicht versehen sind.

Zum Schutz menschlicher Haare vor UV-Strahlen können die erfindungsgemäßen Lichtschutzmittel der Formel I in Shampoos, Lotionen, Gelen, Haarsprays, Aerosol-Schaumcremes oder Emulsionen in Konzentrationen von 0,1 bis 10 Gew.-%, bevorzugt 1 bis 7 Gew.-% eingearbeitet werden. Die jeweiligen Formulierungen können dabei u.a. zum Waschen, Färben sowie zum Frisieren der Haare verwendet werden.

Die erfindungsgemäß zu verwendenden Verbindungen zeichnen sich in der Regel durch ein besonders hohes Absorptionsvermögen im Bereich der UV-A-Strahlung mit scharfer Bandenstruktur aus. Weiterhin sind sie gut in kosmetischen Ölen löslich und lassen sich leicht in kosmetische Formulierungen einarbeiten. Die mit den erfindungsgemäß zu verwendenden Verbindungen hergestellten Emulsionen zeichnen sich besonders durch ihre hohe Stabilität und die damit hergestellten Zubereitungen durch ihr angenehmes Hautgefühl aus.

Die UV-Filterwirkung der erfindungsgemäß zu verwendenden Verbindungen kann auch zur Stabilisierung von Wirk- und Hilfsstoffen in kosmetischen und pharmazeutischen Formulierungen ausgenutzt werden.

Die erfindungsgemäßen Verbindungen eignen sich auch in hervorragender Weise zum Stabilisieren von organischen Materialien gegen die Einwirkung von Licht, Sauerstoff und Wärme.

Als Kunststoffe, die durch die erfindungsgemäßen Verbindungen I stabilisiert werden können, seien beispielsweise genannt:
Polymere von Mono- und Diolefinen, wie z.B. Polyethylen niedriger oder hoher Dichte, Polypropylen, lineares Polybuten-1, Polyisopren, Polybutadien sowie Copolymerisate von Mono- oder Diolefinen oder Mischungen der genannten Polymeren;
Copolymerisate von Mono- oder Diolefinen mit anderen Vinylmonomeren, wie z.B. Ethylen-Alkylacrylat-Copolymere, Ethylen-Alkylmethacrylat-Copolymere, Ethylen-Vinylacetat-Copolymere oder Ethylen-Acrylsäure-Copolymere;
Polystyrol sowie Copolymere von Styrol oder α-Methylstyrol mit Dienen und/oder Acrylderivaten, wie z.B. Styrol-Butadien, Styrol-Acrylnitril (SAN), Styrol-Ethylmethacrylat, Styrol-Butadien-Ethylacrylat, Styrol-Acrylnitril-Methacrylat, AcrylnitrilButadien-Styrol (ABS) oder Methylmethacrylat-Butadien-Styrol (MBS);
Halogenhaltige Polymere, wie z.B. Polyvinylchlorid, Polyvinylfluorid, Polyvinylidenfluorid sowie deren Copolymere;
Polymere, die sich von α,β-ungesättigten Säuren und deren Derivaten ableiten, wie Polyacrylate, Polymethacrylate, Polyacrylamide und Polyacrylnitrile;
Polymere, die sich von ungesättigten Alkoholen und Aminen bzw. von deren Acrylderivaten oder Acetalen ableiten, z.B. Polyvinylalkohol und Polyvinylacetat;
Polyurethane, Polyamide, Polyharnstoffe, Polyphenylenether, Polyester, Polycarbonate, Polyoxymethylene, Polysulfone, Polyethersulfone und Polyetherketone.

Weiterhin können mit den erfindungsgemäßen Verbindungen I Lacküberzüge stabilisiert werden, z.B. Industrielackierungen. Unter diesen sind Einbrennlackierungen, unter diesen wiederum Fahrzeuglackierungen, vorzugsweise Zweischichtlackierungen, besonders hervorzuheben.

Die erfindungsgemäßen Verbindungen I können in fester oder gelöster Form dem Lack zugesetzt werden. Ihre gute Löslichkeit in Lacksystemen ist dabei von besonderem Vorteil.

Bevorzugt werden die erfindungsgemäßen Verbindungen I zum Stabilisieren von Polyolefinen, insbesondere von Polyethylen, von Polycarbonaten, von Polyamiden, von Polyestern, von Polystyrol, von ABS und von Polyurethanen verwendet. Insbesondere können auch Folien aus den genannten Kunststoffen stabilisiert werden.

Für diese Anwendungsbereiche werden die Verbindungen in Konzentrationen von 0,01 bis 5 Gew.-%, bezogen auf die Menge des Kunststoffs, eingesetzt, bevorzugt in einer Konzentration von 0,02 bis 2 Gew.-%. Die Kombination mit anderen Stabilisatoren, beispielsweise Antioxidantien, Metalldesaktivatoren oder anderen Lichtschutzmitteln sowie mit antistatischen oder flammhemmenden Mitteln, ist oft vorteilhaft. Besonders wichtige Costabilisatoren sind beispielsweise sterisch gehinderte Phenole sowie Phosphite, Phosphonite, Amine und Schwefelverbindungen.

Als geeignete Costabilisatoren kommen z.B. in Betracht:
Phenolische Antioxidationsmittel wie
   2,6-Di-tert.-butyl-4-methylphenol,
   n-Octadecyl-β-(3,5-di-tert.-butyl-4-hydroxyphenol)-propionat,
   1,1,3-Tris-(2-methyl-4-hydroxy-5-tert.-butylphenyl)-butan,
   1,3,5-Trimethyl-2,4,6-tris-(3,5-di-tert.-butyl-4-hydroxybenzyl)-benzol,
   1,3,5-Tris-(3,5-di-tert.-butyl-4-hydroxybenzyl)-isocyanurat,
   1,3,5-Tris-[β-(3,5-di-tert.-butyl-4-hydroxyphenyl)-propionylethyl]-isocyanurat,
   1,3,5-Tris-(2,6-di-methyl-3-hydroxy-4-tert.-butylbenzyl)-isocyanurat und
   Pentaerythrit-tetrakis-[β-(3,5-di-tert.-butyl-4-hydroxy)-propionat],
phosphorhaltige Antioxidantien wie
   Tris-(nonylphenyl)-phosphit, Distearylpentaerythritphosphit,
   Tris-(2,4-di-tert.-butyl-phenyl)-phosphit,
   Tris-(2-tert.-butyl-4-methylphenyl)-phosphit,
   Bis-(2,4-di-tert.-butylphenyl)-pentaerythritdiphosphit und
   Tetrakis-(2,4-di-tert.-butylphenyl)-4,4'-biphenylendiphosphit,
schwefelhaltige Antioxidantien wie
   Dilaurylthiodipropionat,
   Dimyristylthiodipropionat,
   Distearylthiodipropionat,
   Pentaerythrittetrakis-(β-laurylthiopropionat) und
   Pentaerythrittetrakis-(β-hexylthiopropionat),
sterisch gehinderte Amine wie
   Bis-(2,2,6,6-tetramethylpiperidyl)-sebacat,
   Bis-(1,2,2,6,6-pentamethylpiperidyl)-sebacat,
   Bis-(1,2,2,6,6-pentamethylpiperidyl)-ester,
   N,N'-Bis(formyl)-bis(2,2,6,6-tetramethyl-4-piperidyl)-1,6-hexandiamin,
   das Kondensationsprodukt von
   1-Hydroxy-2,2,6,6-tetramethyl-4-hydroxypiperidin und Bernsteinsäure,
   das Kondensationsprodukt von
   N,N'-(2,2,6,6-Tetramethylpiperidyl)-hexamethylendiamin und 4-tert.-Octylamino-2,6-dichlor,1,3,5-s-triazin,
   Poly-[3-(Eicosyl/Tetracosyl)-1-(2,2,6,6-tetramethylpiperidin-4-yl)-pyrrolidin-2,5-dion],
   Tris-(2,2,6,6-Tetramethylpiperidyl)-nitrilotriacetat, Tetrakis-(2,2,6,6-tetramethyl-4-piperidyl)-1,2,3,4-butantetracarbonsäure,
   1,1'-(1,2-Ethandiyl)-bis-(3,3,5,5-tetramethylpiperazinon), die Kondensationsprodukte von
   4-Amino-2,2,6,6-tetramethylpiperidinen und Tetramethylolacetylendiharnstoffen sowie
2-(2'-Hydroxyphenyl)-benztriazole,
   2-Hydroxybenzophenone,
   Arylester von Hydroxybenzoesäuren
   α-Cyanozimtsäurederivate
   Nickelverbindungen oder
   Oxalsäuredianilide.

Zur Vermischung der erfindungsgemäßen Verbindungen I, vor allem mit Kunststoffen, können alle bekannten Vorrichtungen und Methoden zum Einmischen von Stabilisierungsmitteln oder anderen Zusätzen in Polymere angewandt werden.

In den folgenden Beispielen wird die Herstellung und Verwendung näher erläutert.

### Beispiele

### Beispiel 1

20,5 g (0,1 mol) 2,2-Bismethylmercapto-1-cyanoacrylsäuremethylester, (hergestellt gemäß den Angaben von CH 601984) und 12,5 g (0,1 mol) 2-Mercaptoanilin werden in 100 ml Ethanol bei Raumtemperatur über Nacht gerührt. Man saugt von ausgefallenen Niederschlag ab, wäscht mit Ethanol und Aceton nach und trocknet bei 200 mbar und 100°C. Ausbeute: 20,2 g (87 % d.Th.) weißes Pulver.

Die Absorptionseigenschaften sind in Tabelle 1 aufgeführt. Weitere Beispiele 2 bis 23 gemäß Tabellen 1, 2 und 3 werden analog erhalten. Substituenten in den aromatischen Ringen können auch nachträglich eingeführt werden.

Dazu werden z.B. 5,8 g (250 mmol) der Verbindung des Beispiels 1 in 50 Dimethylformamid gelöst, mit 4,1 g (250 mmol) Bromhexan und 3,45 g (250 mmol) Kaliumcarbonat versetzt und die Mischung 1 h unter Rückflußkühlung zum Sieden erhitzt. Dann gibt man 50 ml Wasser zu, wodurch das Produkt ausfällt. Die so erhaltene Verbindung ist aus Tabelle 2 als Beispiel 17 ersichtlich. Man saugt ab, wäscht mit Wasser nach und trocknet bei Raumtemperatur unter vermindertem Druck (200 mbar). Ausbeute: 5 g hellgelbes Pulver (63 % d.Th.).

**Tabelle 1**

| | | | | | |
|---|---|---|---|---|---|
| 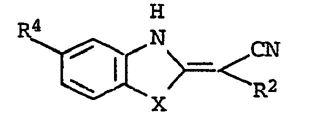 | | | | | |

| | R⁴ | X | R² | λₘₐₓ | E¹₁ |
|---|---|---|---|---|---|
| 1 | H | S | COOCH₃ | 336 | 1723 |
| 2 | H | NH | CN | 314 | 2297 |
| 3 | H | NH | COOCH₃ | 316 | 1986 |
| 4 | OCH₃ | NH | CN | 326 | 1784 |
| 5 | OCH₃ | NH | COOCH₃ | 328 | 1601 |
| 6 | COOC₂H₅ | NH | CN | 334 | 821 |
| 7 | COOC₂H₅ | NH | COOCH₃ | 332 | 952 |
| 8 | CH₃ | NH | CN | 318 | 2055 |
| 9 | CH₃ | NH | COOCH₃ | 320 | 1854 |
| 10 | H | O | CN | 308 | 1844 |
| 11 | H | O | COOCH₃ | 310 | 1778 |
| 12 | H | S | CN | 334 | 1776 |

**Tabelle 2**

| | | | | |
|---|---|---|---|---|
| 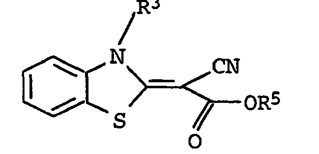 | | | | |

| | R³ | R⁵ | λₘₐₓ | E¹₁ |
|---|---|---|---|---|
| 13 | CH₃ | 2-Ethyl-hexyl | 340 | 1150 |
| 14 | CH₃ | 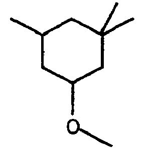 | 340 | 1090 |
| 15 | n-C₆H₁₃ | 2-Ethyl-hexyl | 340 | 830 |
| 16 | n-C₆H₁₃ | 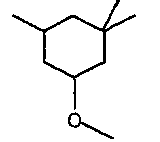 | 340 | 790 |
| 17 | n-C₆H₁₃ | CH₃ | 340 | 1200 |
| 18 | n-C₄H₉ | n-C₄H₉ | 340 | 1250 |

Die Verbindung des Beispiels 18 wurde wie folgt erhalten:
0,1 mol Mercaptoanilin und 0,1 mol 2,2-Bismercapto-1-cyanacrylsäurebutylester wurden in 100 ml Ethanol auf 80°C erhitzt. Anschließend kühlte man auf 0°C ab und saugte das ausgefallene Produkt ab. Nach Auswaschen mit Essigester und Trocknen bei 200 mbar und 60°C erhielt man 20,5 g weiße Kristalle (75 % d. Th.).

Eine Mischung aus 0,1 mol des Reaktionsproduktes, 0,1 mol Kaliumcarbonat und 0,11 mol Butylbromid in 100 ml Dimethylformamid wurden 6 Stunden bei 100°C gerührt. Danach kühlte man auf Raumtemperatur ab und saugte vom ausgefallenen Salz ab. Das Filtrat wurde mit 200 ml Wasser verdünnt und dreimal mit jeweils 100 ml Dichlormethan extrahiert. Die organischen Phasen wurden vereinigt, über Natriumsulfat getrocknet und eingeengt. Das erhaltene gelbe Öl wurde an Kieselgel mit Cyclohexan/Essigester 4:1 chromatographiert. Aus der zweiten Fraktion wurden 6 g (d.s. 18 % d. Th.) der Verbindung 18 als schwach gelbe Kristalle isoliert.

**Tabelle 3**

| | Formel | λₘₐₓ | E¹₁ |
|---|---|---|---|
| 19 | 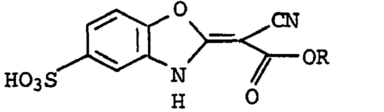 | 312 | 1125 |
| 20 | 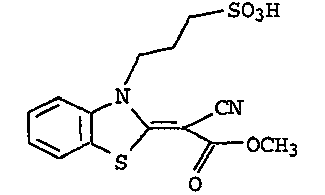 | 340 | 1200 |
| 21 | 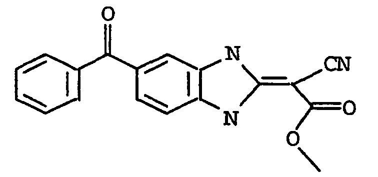 | 348 | 740 |
| 22 | 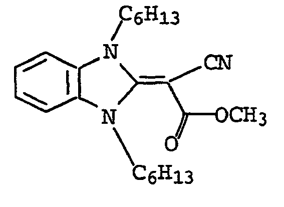 | 325 | 970 |
| 23 | 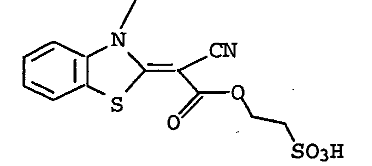 | 340 | 1160 |
| 24 | 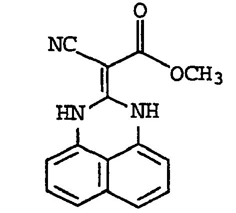 | 296 | 1503 |

### Anwendungsbeispiele

### Beispiel 25

### Standardisierte Methode zur Bestimmung der Photostabilität (Suntest)

Eine 5 Gew.-%ige alkoholische Lösung des zu prüfenden Lichtschutzmittels wird mittels einer Eppendorfpipette (20 µl) auf die Auffräsung eines Glasplättchens aufgetragen. Durch die Anwesenheit des Alkohols verteilt sich die Lösung gleichmäßig auf der aufgerauten Glasoberfläche. Die aufgetragene Menge entspricht der Menge an Lichtschutzmittel, die in Sonnencremes zur Erreichung eines mittleren Lichtschutzfaktors benötigt wird. Bei der Prüfung werden jeweils 4 Glasplättchen bestrahlt. Die Abdampfzeit und die Bestrahlung betragen je 30 Minuten. Die Glasplättchen werden während des Bestrahlens durch eine Wasserkühlung, die sich am Boden des Suntestgeräte befindet, leicht gekühlt. Die Temperatur innerhalb des Suntest Gerätes beträgt während der Bestrahlung 40°C. Nachdem die Proben bestrahlt worden sind, werden sie mit Ethanol in einen dunklen 50 ml Meßkolben gewaschen und mit dem Photometer vermessen. Die Blindproben werden ebenso auf Glasplättchen aufgetragen und 30 Minuten bei Raumtemperatur abgedampft. Wie die anderen Proben werden sie mit Ethanol abgewaschen und auf 100 ml verdünnt und vermessen.

Allgemeine Vorschrift zur Herstellung von Emulsionen für kosmetische Zwecke

Alle öllöslichen Bestandteile werden in einem Rührkessel auf 85°C erwärmt. Wenn alle Bestandteile geschmolzen sind, bzw. als Flüssigphase vorliegen, wird die Wasserphase unter Homogenisieren eingearbeitet. Unter Rühren wird die Emulsion auf ca. 40°C abgekühlt, parfümiert, homogenisiert und dann unter ständigem Rühren auf 25°C abgekühlt.

### Zubereitungen

### Beispiel 26

### Zusammensetzung für die Lippenpflege

### Massengehalt (Gew.-%)

| ad 100 | Eucerinum anhydricum |
|---|---|
| 10,00 | Glycerin |
| 10,00 | Titanium Dioxid, mikronisiert |
| 5,00 | Verbindung Nr. 18 der Tabelle 2 |
| 8,00 | Octyl Methoxycinnamat |
| 5,00 | Zink Oxid |
| 4,00 | Castoröl |
| 4,00 | Pentaerythrithil Stearat/caprat/Caprylat Adipat |
| 3,00 | Glyceryl Stearat SE |
| 2,00 | Bienenwachs |
| 2,00 | Microkristallines Wachs |
| 2,00 | Quaternium-18 Bentonit |
| 1,50 | PEG-45/Dodecyl Glycol Copolymer |

### Beispiel 27

### Zusammensetzung für Sunblocker mit Mikropigmenten

### Massengehalt (Gew.-%)

| ad 100 | Wasser |
|---|---|
| 10,00 | Octyl Methoxcinnamat |
| 6,00 | PEG-7-Hydrogenated Castor Öl |
| 6,00 | Titanium Dioxid, mikronisiert |
| 5,00 | Verbindung Nr. 18 der Tabelle 2 |
| 5,00 | Mineral Öl |
| 5,00 | Isoamyl p-Methoxycinnamat |
| 5,00 | Propylen Glycol |
| 3,00 | Jojoba Öl |
| 3,00 | 4-Methylbenzyliden Campher |
| 2,00 | PEG-45/Dodecyl Glycol Copolymer |
| 1,00 | Dimethicon |
| 0,50 | PEG-40-Hydrogenated Castor Öl |
| 0,50 | Tocopheryl Acetat |
| 0,50 | Phenoxyethanol |
| 0,20 | EDTA |

### Beispiel 28

### Fettfreies Gel

### Massengehalt (Gew.-%)

| ad 100 | Wasser |
|---|---|
| 8,00 | Octyl Methoxycinnamat |
| 7,00 | Titanium Dioxid, mikronisiert |
| 5,00 | Verbindung Nr. 18 der Tabelle 2 |
| 5,00 | Glycerin |
| 5,00 | PEG-25 PABA |
| 1,00 | 4-Methylbenzyliden Campher |
| 0,40 | Acrylate C10-C30 Alkyl Acrylat Crosspolymer |
| 0,30 | Imidazolidinyl Urea |
| 0,25 | Hydroxyethyl Cellulose |
| 0,25 | Sodium Methylparaben |
| 0,20 | Disodium EDTA |
| 0,15 | Fragrance |
| 0,15 | Sodium Propylparaben |
| 0,10 | Sodium Hydroxid |

### Beispiel 29

### Sonnencreme (LSF 20)

### Massengehalt (Gew.-%)

| ad 100 | Wasser |
|---|---|
| 8,00 | Octyl Methoxycinnamat |
| 8,00 | Titanium Dioxid, mikronisiert |
| 6,00 | PEG-7-Hydrogenated Castor Öl |
| 5,00 | Verbindung Nr. 18 der Tabelle 2 |
| 6,00 | Mineral Öl |
| 5,00 | Zink Oxid |
| 5,00 | Isopropyl Palmitat |
| 0,30 | Imidazolidinyl Urea |
| 3,00 | Jojoba Öl |
| 2,00 | PEG-45/Dodecyl Glycol Copolymer |
| 1,00 | 4-Methylbenzyliden Campher |
| 0,60 | Magnesium Stearat |
| 0,50 | Tocopheryl Acetat |
| 0,25 | Methylparaben |
| 0,20 | Disodium EDTA |
| 0,15 | Propylparaben |

### Beispiel 30

### Sonnencreme wasserfest

### Massengehalt (Gew.-%)

| ad 100 | Wasser |
|---|---|
| 8,00 | Octyl Methoxycinnamat |
| 5,00 | PEG-7-Hydrogenated Castor Öl |
| 5,00 | Propylene Glycol |
| 4,00 | Isopropyl Palmitat |
| 4,00 | Caprylic/Capric Triglycerid |
| 5,00 | Verbindung Nr. 18 der Tabelle 2 |
| 4,00 | Glycerin |
| 3,00 | Jojoba Öl |
| 2,00 | 4-Methylbenzyliden Campher |
| 2,00 | Titanium Dioxid, mikronisiert |
| 1,50 | PEG-45/Dodecyl Glycol Copolymer |
| 1,50 | Dimethicon |
| 0,70 | Magnesium Sulfat |
| 0,50 | Magnesium Stearat |
| 0,15 | Fragrance |

### Beispiel 31

### Sonnenmilch (LSF 6)

### Massengehalt (Gew.-%)

| ad 100 | Wasser |
|---|---|
| 10,00 | Mineral Öl |
| 6,00 | PEG-7-Hydrogenated Castor Öl |
| 5,00 | Isopropyl Palmitat |
| 3,50 | Octyl Methoxycinnamat |
| 5,00 | Verbindung Nr. 18 der Tabelle 2 |
| 3,00 | Caprylic/Capric Triglycerid |
| 3,00 | Jojoba Öl |
| 2,00 | PEG-45/Dodecyl Glycol Copolymer |
| 0,70 | Magnesium Sulfat |
| 0,60 | Magnesium Stearat |
| 0,50 | Tocopheryl Acetat |
| 3,00 | Glycerin |
| 0,25 | Methylparaben |
| 0,15 | Propylparaben |
| 0,05 | Tocopherol |

## Patentansprüche

1. Verwendung von verbindungen der Formel I 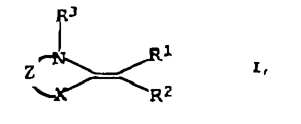 in der
R¹ und R² gleiche oder verschiedene, Elektronen anziehende Reste, ausgewählt aus der Gruppe bestehend aus Cyan, Alkyl- oder Arylcarbonyl, Alkyloxy- oder Aryloxycarbonyl und gegebenenfalls substituiertem Aminocarbonyl,
R³ ein Wasserstoffatom, einen C₁-C₂₀-Alkylrest oder C₃-C₂₀ Cycloalkylrest oder einen Rest der Formel -CH₂-CH₂-SO₃⁻ M⁺ bedeutet, wobei M⁻ für ein Kation steht,
X den zweiwertigen Rest des Sauerstoffs, Schwefels oder den Rest 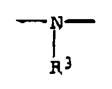 bedeutet, wobei R³ die oben genannte Bedeutung hat,
Z die zweiwertigen Reste der Formeln II oder III bedeutet, die mit dem Rest der Formel I ein anelliertes System bilden 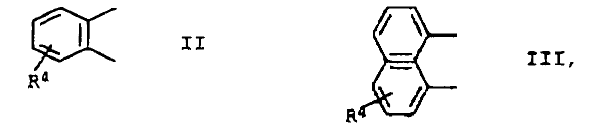 wobei R⁴ ein oder mehrfach an den Benzyliden- I bzw. Naphthylidenring II gebunden sein kann und Wasserstoff, Alkyl, Cycloalkyl, Alkyloxy, Cycloalkyloxy, Alkoxycarbonyl. Mono- oder Dialkylaminocarbonyl, Alkylamino, Dialkylamino, alle mit jeweils bis zu 20 C-Atomen, sowie Cyan, Amino und SO₃⁻M⁺ bedeuten, wobei M⁺ für ein Kation steht,
als photostabile UV-Filter in kosmetischen und pharmazeutischen Zubereitungen zum Schutz der menschlichen Haut und menschlicher Haare gegen Sonnenstrahlen, allein oder zusammen mit an sich für kosmetischen und pharmazeutischen Zubereitungen bekannten, im UV-Bereich absorbierenden Verbindungen.

2. Verwendung von Verbindungen der Formel I gemäß Anspruch 1, **dadurch gekennzeichnet, daß** Z den Rest der Formel II bedeutest.

3. Verwendung von Verbindungen der Formel I gemäß Anspruch 1, **dadurch gekennzeichnet, daß** R¹ Cyan oder Alkyloxycarbonyl mit 2 bis 12 C-Atomen, R² Wasserstoff, R³ Wasserstoff oder Methyl und R⁴ C₁-C₈ Alkyl, C₁-C₈-Alkoxy oder SO₃⁻M⁺ bedeuten, wobei M⁺ für ein Kation steht, ausgewählt aus der Gruppe bestehend aus H⁺, gegebenenfalls substituiertem Ammonium und Metall-kationen.

4. Verwendung von Verbindungen der Formel I gemäß Anspruch 1 als Lichtschutzmittel zur Stabilisierung von Kunststoffen.

5. Lichtschutzmittel enthaltende kosmetische Zubereitungen zum Schutz der menschlichen Epidermis oder der menschlichen Haare gegen UV-Licht im Bereich von 280 bis 400 nm, **dadurch gekennzeichnet, daß** sie in einem kosmetisch geeigneten Träger, allein oder zusammen mit an sich für kosmetische und pharmazeutische Zubereitungen bekannten, im UV-Bereich absorbierenden Verbindungen, Verbindungen der Formel I enthalten 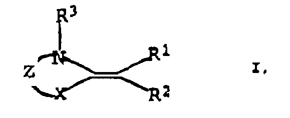 in der
R¹ und R² gleiche oder verschiedene, Elektronen anziehende Reste, ausgewählt aus der Gruppe bestehend aus Cyan, Alkyl- oder Arylcarbonyl, Alkyloxy- oder Aryloxycarbonyl und gegebenenfalls substituiertem Aminocarbonyl,
R³ ein wasserstoffatom, einen C₁-C₂₀-Alkylrest oder C₃-C₂₀ Cycloalkylrest oder einen Rest der Formel -CH₂-CH₂-SO₃- M⁺ bedeutet, wobei M⁺ für ein Metallkation steht,
X den zweiwertigen Rest des Sauerstoffs, Schwefels oder den Rest 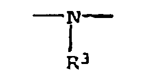 bedeutet, wobei R³ die oben genannte Bedeutung hat
Z die zweiwertigen Reste der Formeln II oder III bedeutet, die mit dem Rest der Formel I ein anelliertes System bilden 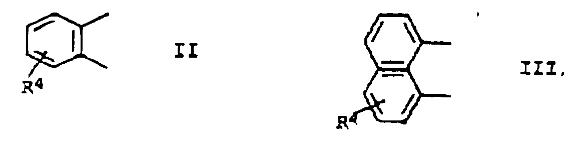
wobei R⁴ ein oder mehrfach an den Benzyliden- I bzw. Naphthylidenring II gebunden sein kann und Wasserstoff, Alkyl, Cycloalkyl, Alkyloxy, Cycloalkyloxy, Alkoxycarbonyl, Mono- oder Dialkylaminocarbonyl, Alkylamino, Dialkylamino, alle mit jeweils bis zu 20 C-Atomen, sowie Cyan, Amino und SO₃-M⁺ bedeuten, wobei M⁺ für ein Metallkation steht.

6. Lichtschutzmittel enthaltende kosmetische Zubereitungen gemäß Anspruch 5, **dadurch gekennzeichnet, daß** sie Verbindungen der Formel I enthalten, in der Z den Rest der Formel II bedeutet.

7. Lichtschutzmittel enthaltende kosmetische Zubereitungen gemäß Anspruch 5, **dadurch gekennzeichnet, daß** sie Verbindungen der Formel I enthalten, in der R¹ Cyan oder Alkyloxycarbonyl mit 2 bis 12 C-Atomen, R² Wasserstoff, R³ Wasserstoff oder Methyl und R⁴ C₁-C₈ Alkyl, C₁-C₈-Alkoxy oder SO₃⁻M⁺ bedeuten, wobei M⁺ für ein Kation steht.

8. Als Lichtschutzmittel geeignete neue Verbindungen der Formel V 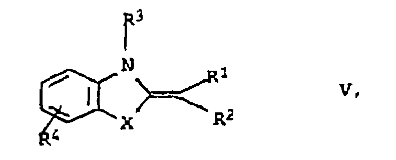 in der X den zweiwertigen Rest des Sauerstoffs, Schwefels oder den Rest 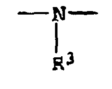 bedeutet, und R¹ und R² unabhängig voneinander CN oder Alkoxycarbonyl mit 2 bis 10 C-Atomen, das durch einen Rest (-CH₂)ₙ-SO₃-M⁺ substituiert sein kann, R³ Wasserstoff, Methyl oder -(CH₂)ₙ-SO₃⁻M⁺ und R⁴ Wasserstoff oder einen oder mehrere gleiche oder verschiedene Reste C₁- bis C₈-Alkyl. C₁- bis C₈-Alkoxy oder den Rest -SO₃-M⁺ bedeutet, wobei jeweils n die Zahlen 2 oder 3 und M⁺ ein Kation bedeutet, ausgewählt aus der Gruppe bestehend aus 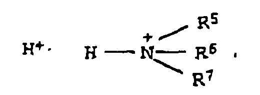 in der R⁵, R⁶ und R⁷ für Wasserstoff oder niedermolekulares Alkyl oder Hydroxyalkyl steht, und Metallkationen,
mit der Maßgabe, daß die Verbindung der Formel V mindestens einen Rest -(CH₂)ₙSO₃⁻M⁺ oder -SO₃⁻M⁺ aufweist.

## Claims

1. The use of compounds of the formula I 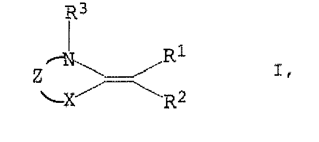 in which
R¹ and R² are identical or different, electron-withdrawing radicals chosen from the group consisting of cyano, alkyl- or arylcarbonyl, alkyloxy- or aryloxycarbonyl and optionally substituted aminocarbonyl,
R³ is a hydrogen atom, a C₁-C₂₀-alkyl radical or a C₃-C₂₀-cycloalkyl radical or a radical of the formula -CH₂-CH₂-SO₃⁻ M⁺, where M⁺ is a cation,
X is the divalent radical of oxygen, sulfur or the radical 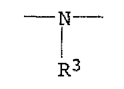 where R³ is as defined above,
Z is the divalent radical of the formula II or III, which forms a fused system with the radical of the formula I 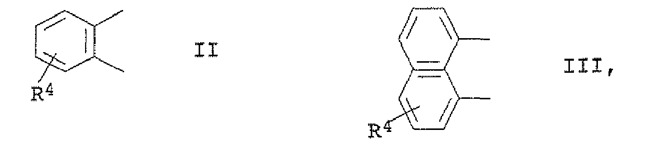
where R⁴ can be bonded one or more times to the benzylidene ring I or naphthylidene ring II, and is hydrogen, alkyl, cycloalkyl, alkyloxy, cycloalkyloxy, alkoxycarbonyl, mono- or dialkylaminocarbonyl, alkylamino, dialkylamino, each having up to 20 carbon atoms, and also cyano, amino and SO₃-M⁺, where M⁺ is a cation,
as photostable UV filters in cosmetic and pharmaceutical preparations to protect human skin and human hair against solar rays, alone or together with compounds which absorb in the UV region and are known per se for cosmetic and pharmaceutical preparations.

2. The use of compounds of the formula I as claimed in claim 1, wherein Z is the radical of the formula II.

3. The use of compounds of the formula I as claimed in claim 1, wherein R¹ is cyano or alkyloxycarbonyl having from 2 to 12 carbon atoms, R² is hydrogen, R³ is hydrogen or methyl, and R⁴ is C₁-C₈-alkyl, C₁-C₈-alkoxy or SO₃⁻M⁺, where M⁺ is a cation chosen from the group consisting of H⁺, optionally substituted ammonium, and metal cations.

4. The use of compounds of the formula I as claimed in claim 1 as light protection agents for stabilizing plastics.

5. A cosmetic preparation comprising light protection agents, for protecting the human epidermis or human hair from UV light in the range from 280 to 400 nm, which comprises, in a cosmetically suitable carrier, alone or together with compounds which absorb in the UV region and are known per se for cosmetic and pharmaceutical preparations, compounds of the formula I 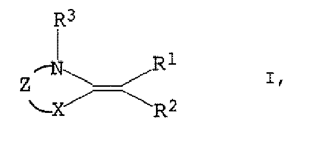 in which
R¹ and R² are identical or different, electron-withdrawing radicals chosen from the group consisting of cyano, alkyl- or arylcarbonyl, alkyloxy- or aryloxycarbonyl and optionally substituted aminocarbonyl,
R³ is a hydrogen atom, a C₁-C₂₀-alkyl radical or a C₃-C₂₀-cycloalkyl radical or a radical of the formula -CH₂-CH₂-SO₃- M⁺, where M⁺ is a metal cation,
X is the divalent radical of oxygen, sulfur or the radical 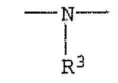 where R³ is as defined above,
Z is the divalent radical of the formula II or III, which forms a fused system with the radical of the formula I 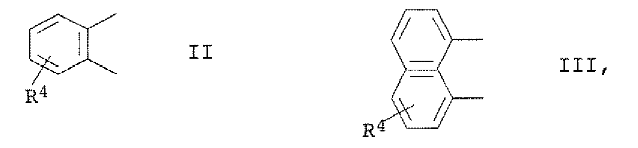
where R⁴ can be bonded one or more times to the benzylidene ring I or naphthylidene ring II, and is hydrogen, alkyl, cycloalkyl, alkyloxy, cycloalkyloxy, alkoxycarbonyl, mono- or dialkylaminocarbonyl, alkylamino, dialkylamino, each having up to 20 carbon atoms, and also cyano, amino and SO₃⁻M⁺, where M⁺ is a metal cation.

6. The cosmetic preparation comprising light protection agents as claimed in claim 5, which comprises compounds of the formula I in which Z is the radical of the formula II.

7. The cosmetic preparation comprising light protection agents as claimed in claim 5, which comprises compounds of the formula I in which R¹ is cyano or alkyloxycarbonyl having from 2 to 12 carbon atoms, R² is hydrogen, R³ is hydrogen or methyl, and R⁴ is C₁-C₈-alkyl, C₁-C₈-alkoxy or SO₃-M⁺, where M⁺ is a cation.

8. A novel compound, suitable as light protection agent, of the formula V 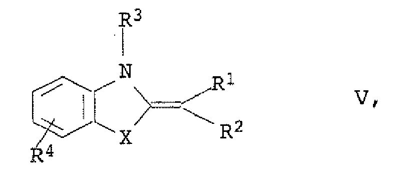 in which X is the divalent radical of oxygen, sulfur or the radical 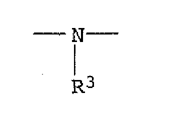 and R¹ and R² independently of one another are CN or alkoxycarbonyl having from 2 to 10 carbon atoms which can be substituted by a radical -(CH₂)ₙ-SO₃⁻M+, R³ is hydrogen, methyl or -(CH₂)ₙ-SO₃-M⁺, and R⁴ is hydrogen or one or more identical or different radicals C₁- to C₈-alkyl, C₁- to C₈-alkoxy or the radical -SO₃-M⁺, where in each case n is the number 2 or 3 and M⁺ is a cation chosen from the group consisting of 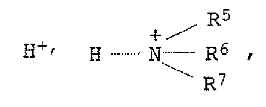 in which R⁵, R⁶ and R⁷ are hydrogen or low molecular weight alkyl or hydroxyalkyl, and metal cations,
with the proviso that the compound of the formula V has at least one radical -(CH₂)ₙSO₃⁻M⁺ or -SO₃⁻M⁺.

## Revendications

1. Utilisation de composés d'après la formule I 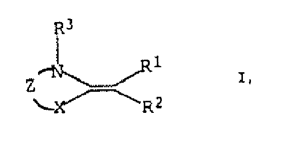 où
R¹ et R² représentent des radicaux identiques ou différents attirant des électrons, sélectionnés dans le groupe constitué de cyan, d'alkylcarbonyle ou d'arylcarbonyle, d'alkyloxycarbonyle ou d'aryloxycarbonyle, et d'aminocarbonyle éventuellement substitué,
R³ représente un atome d'hydrogène, un radical alkyle en C₁-C₂₀, ou un radical cycloalkyle en C₃-C₂₀, ou un radical de formule -CH₂-CH₂-SO₃⁻M⁺, M⁺ désignant un cation,
X représente le radical bivalent de l'oxygène, du souffre, ou bien le radical 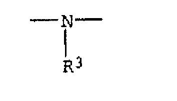 où R³ a la signification indiquée ci-dessus,
Z représente les radicaux bivalents des formules II ou III, qui forment avec le radical de la formule I un système à noyaux condensés 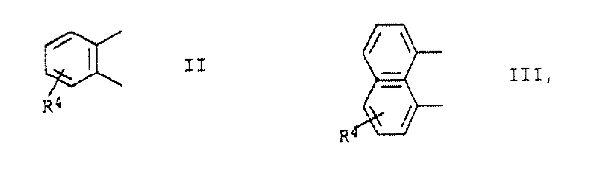
où R⁴ peut être combiné de manière unique ou multiple au noyau de benzylidène I ou au noyau de naphtylidène II, et l'hydrogène, l'alkyle, le cycloalkyle, l'alkyloxy, le cycloalkyloxy, l'alcoxycarbonyle, le mono- ou le dialkylaminocarbonyle, l'alkylamino, le dialkylamino, comportant tous respectivement jusqu'à 20 atomes C chacun, ainsi que du cyan, représentent un amino et du SO₃⁻M⁺, M⁺ désignant un cation,
en tant que filtre UV photostable dans des préparations cosmétiques et pharmaceutiques pour la protection de la peau humaine et des cheveux humains contre les rayons solaires, seul ou conjointement avec des composés absorbants dans la gamme UV, connues par elles-mêmes en tant que préparations cosmétiques et pharmaceutiques.

2. Utilisation de composés d'après la formule I selon la revendication 1, **caractérisée en ce que** Z désigne le radical de la formule II.

3. Utilisation de composés d'après la formule I selon la revendication 1, **caractérisée en ce que** R¹ représente du cyan ou de l'alkyloxycarbonyle avec de 2 à 12 atomes C, R² de l'hydrogène, R³ de l'hydrogène ou du méthyle, et R⁴ un alkyle en C₁-C₈, un alcoxy en C₁-C₈, ou du SO₃⁻M⁺, M⁺ désignant un cation, sélectionné dans le groupe constitué de H⁺, d'ammonium éventuellement substitué et de cations métalliques.

4. Utilisation de composés d'après la formule I selon la revendication 1 en tant que produit de protection contre la lumière pour la stabilisation de matières plastiques.

5. Préparations cosmétiques contenant un produit de protection contre la lumière pour la protection de l'épiderme humain ou des cheveux humains contre la lumière ultraviolette dans la gamme de 280 à 400 nm, **caractérisées en ce qu'**elles contiennent des composés d'après la formule I dans un excipient adapté à un usage cosmétique, seuls ou conjointement avec des composés absorbants dans la gamme UV, connues par elles-mêmes en tant que préparations cosmétiques et pharmaceutiques, 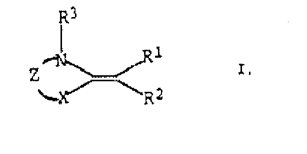 où
R¹ et R² représentent des radicaux identiques ou différents attirant des électrons, sélectionnés dans le groupe constitué de cyan, d'alkylcarbonyle ou d'arylcarbonyle, d'alkyloxycarbonyle ou d'aryloxycarbonyle, et d'aminocarbonyle éventuellement substitué,
R³ représente un atome d'hydrogène, un radical alkyle en C₁-C₂₀, ou un radical cycloalkyle en C₃-C₂₀, ou un radical de formule -CH₂-CH₂-SO₃⁻M⁺, où M⁺ désigne un cation métallique,
X représente le radical bivalent de l'oxygène, du souffre, ou bien le radical 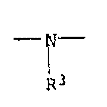 où R³ a la signification indiquée ci-dessus,
Z représente les radicaux bivalents des formules II ou III, qui forment avec le radical de la formule I un système à noyaux condensés 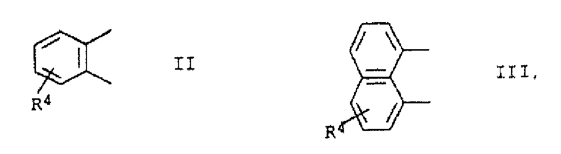
où R⁴ peut être combiné de manière unique ou multiple au noyau de benzylidène I ou au noyau de naphtylidène II, et l'hydrogène, l'alkyle, le cycloalkyle, l'alkyloxy, le cycloalkyloxy, l'alcoxycarbonyle, le mono- ou le dialkylaminocarbonyle, l'alkylamino, le dialkylamino, comportant tous respectivement jusqu'à 20 atomes C chacun, ainsi que du cyan, représentent un amino et du SO₃⁻M⁺, M⁺ désignant un cation métallique,

6. Préparations cosmétiques contenant un produit de protection contre la lumière selon la revendication 5, **caractérisées en ce qu'**elles contiennent des composés d'après la formule I, où Z représente le radical de la formule II.

7. Préparations cosmétiques contenant un produit de protection contre la lumière selon la revendication 5, **caractérisées en ce qu'**elles contiennent des composés d'après la formule I, où R¹ représente du cyan ou de l'alkyloxycarbonyle avec de 2 à 12 atomes C, R² de l'hydrogène, R³ de l'hydrogène ou du méthyle, et R⁴ un alkyle en C₁-C₈, un alcoxy en C₁-C₈, ou du SO₃⁻M⁺, M⁺ désignant un cation.

8. Nouveaux composés adaptés en tant que produit de protection contre la lumière d'après la formule V 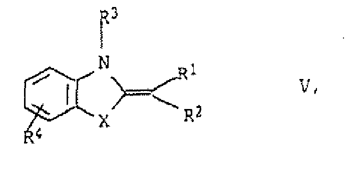 où X représente le radical bivalent de l'hydrogène, du souffre ou le radical 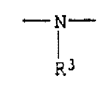 et R¹ et R² représentent indépendamment l'un de l'autre du CN ou de l'alcoxycarbonyle avec de 2 à 10 atomes C, pouvant être substitués par un radical -(CH₂)ₙ-SO₃⁻M⁺, R³ de l'hydrogène, du méthyle, ou du -(CH₂)ₙ-SO₃⁻M⁺, et R⁴ de l'hydrogène ou bien un ou plusieurs radicaux identiques ou différents, un alkyle en C₁-C₈, un alcoxy en C₁-C₈, ou le radical SO₃⁻M⁺, n pouvant prendre les valeurs 2 ou 3, et M⁺ représentant un cation, sélectionné dans le groupe constitué de 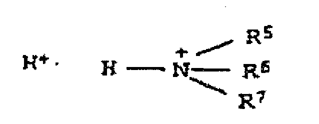 où R⁵, R⁶ et R⁷ désignent de l'hydrogène, ou un alkyle ou un hydroxyalkyle de faible masse moléculaire, et des cations métalliques,
avec la caractéristique que le composé d'après la formule V présente au moins un radical -(CH₂)ₙSO₃⁻M⁺ ou -SO₃⁻M⁺.
